(19) Europäisches Patentamt
European Patent Office
Office européen des brevets

(11) Publication number: 0 196 224
B1

# (12) EUROPEAN PATENT SPECIFICATION

(45) Date of publication of the patent specification: **16.08.89**

(51) Int. Cl.⁴: **C 07 D 471/04** // A61K31/44, (C07D471/04, 235:00, 221:00)

(21) Application number: **86302225.7**

(22) Date of filing: **26.03.86**

(54) **Synthesis of inotropic imidazo[4,5-c]pyridine.**

(30) Priority: **29.03.85 US 718000**

(43) Date of publication of application: **01.10.86 Bulletin 86/40**

(45) Publication of the grant of the patent: **16.08.89 Bulletin 89/33**

(84) Designated Contracting States: **AT BE CH DE FR GB IT LI NL SE**

(56) References cited:
**EP-A- 0 072 926**
**EP-A- 0 079 083**
**EP-A- 0 093 593**

**The file contains technical information submitted after the application was filed and not included in this specification**

(73) Proprietor: **ELI LILLY AND COMPANY, Lilly Corporate Center, Indianapolis Indiana 46285 (US)**

(72) Inventor: **Greene, James Michael, 607 West Ralston Road, Indianapolis, IN 46217 (US)**
Inventor: **Goedde, Jane Ann, 6527 Stearns Hill Drive, Indianapolis, IN 46237 (US)**

(74) Representative: **Tapping, Kenneth George et al, Erl Wood Manor, Windlesham Surrey, GU20 6PH (GB)**

EP 0 196 224 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for preparing 2-[2-methoxy-4-(methylthio)phenyl]-1(3)H-imidazo[4,5-c]pyridine of the formulae

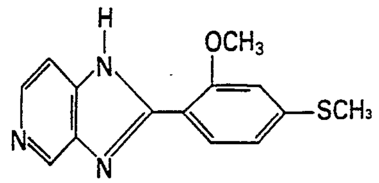

I

or

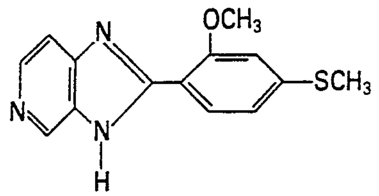

Ia

comprising reacting 3,4-diaminopyridine with 2-methoxy-4-methylthiobenzoic acid in the presence of phosphorus oxychloride and pyridine at a temperature of about 25°C to about 125°C.

All temperatures shall be reported herein as degrees Celsius. All units of measurement are reported in weight units, except for liquids, which are reported in volume units.

The Formulæ I and Ia set forth above represent tautomeric structures. The imidazopyridine having the hydrogen atom on the N-1 nitrogen atom and having the pyridine nitrogen atom at the 5-position is represented by the compound of Formula I and is properly named 2-[2-methoxy-4-(methylthio)phenyl]-1H-imidazo[4,5-c]pyridine. This compound has a corresponding tautomeric form wherein the hydrogen atom is on the N-3 nitrogen atom (Formula Ia) and is named 2-[2-methoxy-4-(methylthio)phenyl]-3H-imidazo [4,5-c]pyridine. As N-unsubstituted compounds, each tautomeric form exists in equilibrium with the other and cannot be prepared or isolated without the presence of the other. For this invention, both forms will be considered together and will be referred to as 2-[2-methoxy-4-(methylthio)-phenyl]-1(3)H-imidazo[4,5-c]pyridine.

The present invention relates to a process for preparing 2-[2-methoxy-4-(methylthio)phenyl]-1(3)H-imidazo[4,5-c]pyridine comprising reacting 3,4-diaminopyridine with 2-methoxy-4-methylthiobenzoic acid in the presence of phosphorus oxychloride ($POCl_3$) and pyridine. This reaction is represented by the following scheme:

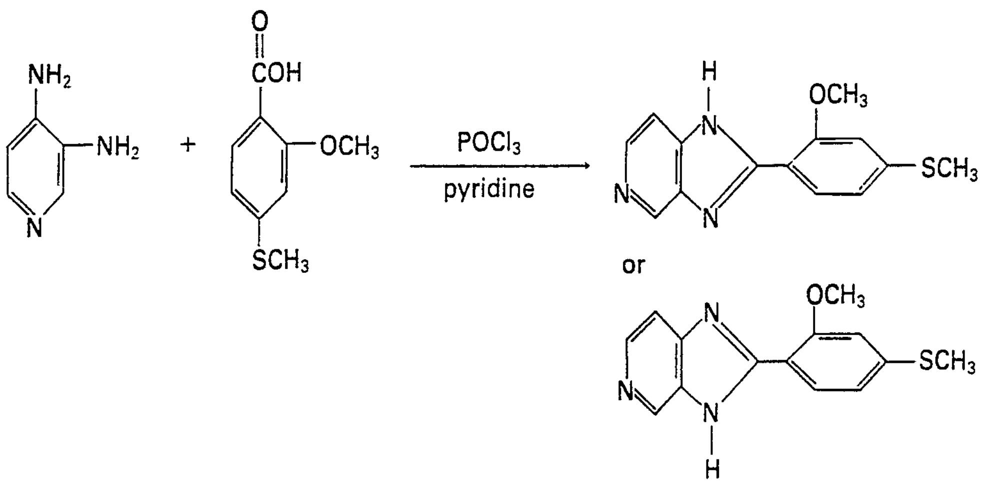


I

Ia

According to the present process, approximately equimolar quantities of 3,4-diaminopyridine and 2-methoxy-4-methylthiobenzoic acid are combined with pyridine in a suitable reaction vessel. The concentration of the starting materials in the pyridine is not critical, but it is preferred to employ a sufficient amount of solvent to keep the two reactants in solution, or a slight excess. Large volumes of solvent are neither necessary nor desirable in the present process.

Phosphorus oxychloride is also present in the present process acting as a means of removing the water produced in the process. During the process two molar equivalents of water are generated for each molar equivalent of the limiting starting material present in the reaction mixture. Accordingly, at least two molar equivalents of phosphorus oxychloride are employed in the present process for each molar equivalent of the limiting starting material. Typically, excess amounts of phosphorus oxychloride are employed. The preferred amount of phosphorus oxychloride for use in the present process is about three molar equivalents. Phosphorus oxychloride also reacts with 2-methoxy-4-methylthiobenzoic acid to provide 2-methoxy-4-methylthiobenzoyl chloride, which then reacts with 3,4-diaminopyridine to give the desired product.

Since phosphorus oxychloride reacts exothermically with water, it is preferred to add the solution of reactants in pyridine slowly to the solution of phosphorus oxychloride in order to better control the accompanying increase in temperature of the reaction. Preferably each solution is heated to a temperature in the range of about 40°C to about 60°C, more specifically at a temperature of about 50°C prior to their combination.

The process of the present invention is substantially complete after about 1 to 24 hours when conducted at a temperature in the range of about

25° C to about 125° C, more preferably from about 50° C to about 80° C.

Once the process of the present invention is substantially complete, the product may be isolated by combining the reaction mixture with a suitable base such as sodium hydroxide at a cold temperature, that is, in the range of about -20° C to about 15° C. The volatile organic constituents are preferably removed by evaporation under vacuum. The product thus prepared is then isolated by vacuum filtration to afford the desired compound in sufficient purity for further synthetic modification without competing adverse side-reactions.

The product of the present process is typically isolated as the free base which is usually hydrated with one equivalent of water. However, the product may be isolated as the hydrochloride acid addition salt, and dried to provide either the anhydrous form or a hydrated form of the molecule. Accordingly, the compounds of formulæ I and Ia prepared by the present process encompass both the free base and the hydrochloric acid salt, in both the hydrated and anhydrous forms.

The present process has been found to provide the desired compounds in high yield and in pure form. The present process is particularly advantageous due to its operability on a large scale thereby making it suitable for an industrial setting. Known procedures using phosphorus oxychloride do not employ pyridine. The reactions wherein pyridine is absent do not operate acceptably on a large scale because of the use of hot phosphorus oxychloride, which oftentimes results in an uncontrollable exotherm and partial loss of reactants. Pyridine permits the easy control of the present process to enable large scale operation. In the prior process, excess phosphorus oxychloride was difficult to remove even under vacuum, and the addition of water is exothermic and uncontrollable. Also, the pyridine employed in the present process neutralizes the hydrochloric acid which is formed. Accordingly, the product is much easier to isolate as compared with the prior process.

The product of the present process, 2-[2-methoxy-4-(methylthio)phenyl]-1(3)H-imidazo-[4,5-c]-pyridine, may be used as an inotropic agent following isolation from the reaction mixture as described above. However, the product is preferably oxidized to the corresponding sulfoxide derivative, 2-[2-methoxy-4-(methylsulfinyl)-phenyl]-1(3)H-imidazo[4,5-c]pyridine, a preferred inotropic agent.

The use of the product of the present process, and its sulfoxide derivative, is known. See, e.g. European Patent Applications 9359379083 and 72926. The compounds are useful as orally effective positive inotropic agents and have minimal effects on blood pressure and heart rate.

While a variety of methods may be employed to oxidize 2-[2-methoxy-4-(methylthio)phenyl]-1(3)H-imidazo[4,5-c]pyridine to 2-[2-methoxy-4-(methylsulfinyl)phenyl]-1(3)H-imidazo[4,5-c]pyridine, the reaction is preferably conducted with aqueous hydrogen peroxide and glacial acetic acid. The reaction is conducted at a temperature in the range of about 0° C to about 50° C for about 1 to about 24 hours, more preferably at a temperature in the range of about 10° C to about 30° C for a period of about 2 to 10 hours. The product may then be economically and conveniently isolated by forcing the product to precipitate from solution by the addition of isopropanol. The product thus isolated is then collected by vacuum filtration and washed as desired to provide the sulfoxide. The sulfoxide thus prepared is finally converted to the hydrochloride salt according to standard procedures to provide the hydrochloride salt of the sulfoxide.

The starting materials employed in the present process are known and either commercially available or readily prepared by procedures well known to one of ordinary skill in the art. For example, 3,4-diaminopyridine may be purchased from Aldrich Chemical Company, Milwaukee, Wisconsin. The compound 2-methoxy-4-methylthiobenzoic acid may be purchased from SSF Dottikon, Dottikon, Switzerland.

The following Examples illustrate specific aspects of the present invention. The Examples are not intended to be limiting to the scope of the present process in any respect and should not be so construed.

The compound prepared by the present process, 2-[2-methoxy-4-(methylthio)phenyl]-1(3)H-imidazo[4,5-c]pyridine, and its sulfoxide form 2-[2-methoxy-4-(methylsulfinyl)phenyl]-1H-imidazo-[4,5-c]pyridine, were characterized by a high performance liquid chromatographic comparison with authentic reference standards. The samples were dissolved in an internal standard solution consisting of 0.7 mg of 2-naphthalene sulfonic acid sodium salt per ml of an elution solvent comprised of 400 parts of acetonitrile, 100 parts of tetrahydrofuran, 21 parts of glacial acetic acid and 1479 parts of water. The column was eluted with the elution solvent listed above containing 2.2 g of 1-heptane sulfonic acid sodium salt. The column employed was a Whatman Partisil PXS 5/25 ODS. The detector had a wavelength of 320 nm, the column flow rate was 1.0 ml/min, the injection volume was 20 µl and the column temperature was ambient. Three samples were assayed for each compound, and the average which was taken was ± 1.9% of the true value at a 95% confidence level.

Example 1

2-[2-Methoxy-4-(methylthio)phenyl]-1(3)H-imidazo[4,5-c]pyridine monohydrate.

A 500 ml 3-neck round bottom flask was charged with 20.81 g (0.191 mol) of 3,4-diaminopyridine (Aldrich Chemical Co., Milwaukee, Wisconsin) and 37.8 g (0.191 mol) of 2-methoxy-4-methylthiobenzoic acid in 260 ml of pyridine. The resulting mixture was heated to approximately 50°C and held at that temperature while a second flask was prepared. A 2 l. 3-neck round bottom flask was charged with 240 ml of

pyridine and 87.72 g (53.3 ml, 0.572 mol) of phosphorus oxychloride. This mixture was heated to about 50°C and the contents of the 500 ml flask were slowly added to the phosphorus oxychloride solution while maintaining the temperature of the reaction mixture below approximately 80°C. The solution became red in color and the temperature was maintained at about 80°C for about 16 hours following addition. The reaction mixture was slowly added to a solution of 230 ml of 25% sodium hydroxide (v:v) at a temperature of about 0°C to about 5°C while maintaining the temperature of the mixture below approximately 20°C. The pH of the mixture was lowered from about 12.5 to about 8.5 with concentrated hydrochloric acid and approximately 500 ml pyridine was evaporated under vacuum at about 60°C. The resulting mixture was stirred at room temperature overnight and 380 ml of water was added. Three hundred and eighty milliliters of solvent were removed under vacuum and 380 ml of water was added to the mixture. The mixture was stirred at room temperature overnight and 400 ml of water was added. The mixture was stirred for 1 hour and filtered. The collected solid was washed twice with 40 ml portions of water and dried under vacuum at about 50°C to provide 37.05 g of 2-[2-methoxy-4-(methylthio)phenyl]-1(3)H-imidazo-[4,5-c]pyridine monohydrate. Yield 67%.

Example 2

2-[2-methoxy-4-(methylthio)phenyl]-1(3)H-imidazo[4,5-c]pyridine monohydrate.

Following the general reaction conditions of Example 1, the reaction mixture was heated for 8 hours at 80°C to provide 39.08 g of the title compound.

Example 3

2-[2-methoxy-4-(methylthio)phenyl]-1(3)H-imidazo[4,5-c]pyridine monohydrate.

Following the general reaction conditions of Example 2, 38.7 g of 2-[2-methoxy-4-(methylthio)phenyl]-1(3)H-imidazo[4,5-c]pyridine monohydrate was obtained.

Example 4

2-[2-methoxy-4-(methylthio)phenyl]-1(3)H-imidazo[4,5-c]pyridine monohydrate.

A 500 ml 3-neck round bottom flask was charged with 10.4 g (0.095 mol) of 3,4-diaminopyridine, 18.9 g (0.095 mol) of 2-methoxy-4-methylthiobenzoic acid and 133.6 ml of pyridine. The resulting solution was heated to approximately 50°C and a second solution was prepared in a 2 l. 3-neck round bottom flask. The second solution consisted of 120 ml of pyridine to which 43.85 g (26.7 ml, 0.286 mol) of phosphorus oxychloride was added dropwise. The resulting mixture was heated to approximately 50°C and the contents of the first reaction vessel were slowly added to the reaction mixture contained within the second vessel. A slight exotherm was observed and a positive nitrogen purge was provided. The resulting reaction mixture was heated at 80°C for 16 hours. The reaction mixture was added slowly to a solution of 115 ml of 25% sodium hydroxide at a temperature of about 0°C to about 5°C while maintaining the temperature of the reaction mixture below approximately 20°C. An additional 45 ml of water and 15 ml of pyridine were added to the reaction mixture. The pH of the solution was adjusted from about 12.3 to about 8 by the dropwise addition of concentrated hydrochloric acid over 1 hour. The reaction mixture was vacuum distilled at about 60°C to provide 222 ml of distillate. Approximately 400 ml of water was added to the reaction mixture which was heated for 1 hour at about 40°C to about 50°C. The reaction mixture was stirred at room temperature overnight and the precipitated solid was collected by vacuum filtration. The resulting solid was washed twice with 20 ml portions of water and dried in a vacuum oven at about 50°C overnight to provide 22.3 g of 2-[2-methoxy-4-(methylthio)phenyl]-1(3)H-imidazo[4,5-c]pyridine monohydrate. Yield 81.1%.

Example 5

2-[2-methoxy-4-(methylthio)phenyl]-1(3)H-imidazo[4,5-c]pyridine monohydrate.

One hundred and twenty liters of dry pyridine were added to a 50 gallon glass-lined reactor under nitrogen. To the pyridine were added 9.4 kg (86.2 mol) of 3,4-diaminopyridine and 17.0 kg (85.9 mol) of 2-methoxy-4-methylthiobenzoic acid. The mixture was stirred at 45°C for 1 hour until a homogeneous slurry was obtained. The mixture was cooled to about 25°C. Dry pyridine (150 l.) was added to a 200 gallon glasslined reactor under a nitrogen atmosphere followed by 39.4 kg (257.5 mol) of phosphorus oxychloride. There was a very slight exotherm. The contents of the reactor were heated to 50°C. The rapidly stirred slurry in the 50 gallon reactor was added to the 200 gallon reactor. The reaction temperature was maintained below about 75°C by controlling the rate of addition. The addition required 20 to 30 minutes. The reactor contents were heated at 75–80°C for eight hours. The reactor was cooled to about 50°C. In a second 200 gallon reactor 104 l. of 25% aqueous sodium hydroxide solution was prepared from 50% sodium hydroxide and water and cooled to 0°C. The warm contents of first 200 gallon reactor were added to the second 200 gallon reactor while maintaining the temperature of the reaction mixture below about 20°C. The addition required 4 hours. After all the material had been added, the temperature was adjusted to about 20°C. The pH of the reaction mixture was adjusted to 7.5 to 8.5 with sodium hydroxide. The reaction mixture was distilled under vacuum [5–10 mm Hg and 50°C pot temperature] until 170 l. of distillate was collected. One hundred and seventy liters of water were added, and distillation was continued until an additional 170 l. of distillate was collected. An addi-

tional 170 l. of water was added to the reaction vessel. The contents of the vessel were checked by G.C. assay and less than 1% by weight of pyridine remained. Another 320 l. of water was added to the vessel. The reactor was stirred for 2 hours at 20° C. The solid was collected by vacuum filtration and washed with 200 l. of water. The wet solid was retourned to the second 200 gallon reactor and slurried with 290 l. of water and 96 l. of pyridine at 50° C for 1 hour. The mixture was cooled to 20° C, and the solid was collected by filtration. The solid was washed with 50 l. of water and air-dried at 70° C to provide 17.8 kg of 2-[2-methoxy-4-(methylthio)phenyl]-1(3)H-imidazo-[4,5-c]pyridine monohydrate. Yield 71.6%.
(200 gallon = 909 l.; 5–10 mmHg = 6.58–13.16 kPa, 50 gallon = 227.25 l.).

Preparation 1

2-[2-methoxy-4-(methylsulfinyl)phenyl]-1(3)H-imidazo[4,5-c]pyridine monochloride monohydrate.

In a 200 gallon glass-lined reactor 99 l. of glacial acetic acid and 17.8 kg (61.5 mol) of 2-[2-methoxy-4-(methylthio)phenyl]-1(3)H-imidazo[4,5-c]pyridine were mixed and cooled to 15° C under nitrogen. Thirty percent hydrogen peroxide (7.12 kg, 62.8 mol) was added slowly. The temperature of the reaction mixture was controlled by external cooling. After 4 hours the cooling was removed, and the reaction mixture was stirred for 20 hours at about 20° C. Isopropanol (260 l.) was added, and the reaction mixture was cooled to about 0–5° C. The reaction mixture was stirred for about 5 hours at about 0–5° C after crystallization occurred. The solid was collected by vacuum filtration and washed with a cold solution of 9 l. of glacial acetic acid and 24 l. of isopropanol. The solid was dried and added to a solution of 58 l. of water and 9 l. of concentrated hydrochloric acid in a stirred portable pot. Two kilograms of decolorizing carbon were added, and the mixture was stirred for 2 hours. The mixture was filtered through a single plate press coated with Celite and backed-up with a Cuno filter. After the aqueous solution was filtered, 435 l. of isopropanol was filtered through the system and crystallization occurred. The reaction mixture was cooled to a temperature of about 0° C to about -5° C with brine and stirred for 6 hours. The solid was collected by filtration, washed with a cold solution of 9 l. of glacial acetic acid and 68 l. of isopropanol and then with 77 l. of isopropanol. The solid was dried in a vacuum drier at about 45° C to about 50° C until the water content was about 5.5% to provide 17.4 kg of 2-[2-methoxy-4-(methylsulfinyl)phenyl]-1(3)H-imidazo[4,5-c]pyridine monohydrochloride monohydrate. Yield 82.8%.

## Claims

1. A process for preparing 2-[2-methoxy-4-(methylthio)phenyl]-1(3)H-imidazo[4,5-c]pyridine of the formulæ

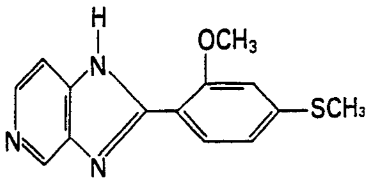

I

or

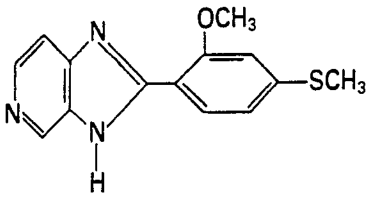

Ia

comprising reacting 3,4-diaminopyridine with 2-methoxy-4-methylthiobenzoic acid in the presence of phosphorus oxychloride and pyridine at a temperature of about 25° C to about 125° C.

2. A process of claim 1 wherein the reaction is conducted at a temperature of about 50° C to about 80° C.

3. A process of claim 1 wherein approximately equimolar quantities of 3,4-diaminopyridine and 2-methoxy-4-methylthiobenzoic acid are employed.

## Patentansprüche

1. Verfahren zur Herstellung von 2-[2-Methoxy-4-(methylthio)phenyl]-1(3)H-imidazo[4,5-c]pyridin der Formeln

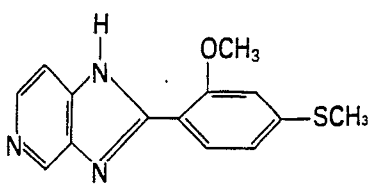

I

oder

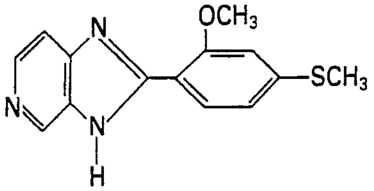

Ia

dadurch gekennzeichnet, daß 3,4-Diaminopyridin mit 2-Methoxy-4-methylthiobenzoesäure in Gegenwart von Phosphoroxychlorid und Pyridin bei einer Temperatur von etwa 25° C bis etwa 125° C umgesetzt wird.

2. Verfahren nach Anspruch 1, dadurch gekennzeichnet, dass die Umsetzung bei einer Temperatur von etwa 50° C bis etwa 80° C durchgeführt wird.

3. Verfahren nach Anspruch 1, dadurch gekennzeichnet, dass etwa äquimolare Mengen von 3,4-Diaminopyridin und 2-Methoxy-4-methylthiobenzoesäure angewandt werden.

**Revendications**

1. Procédé de préparation de la 2-[2-méthoxy-4-(méthylthio)phényl]-1(3)H-imidazo[4,5-c]pyridine répondant aux formules:

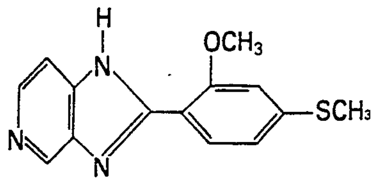

I

ou

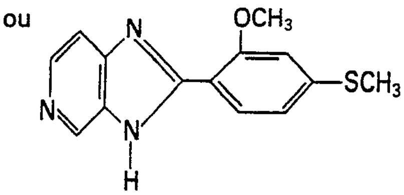

Ia

caractérisé en ce qu'il comprend les étapes qui consistent à faire réagir la 3,4-diaminopyridine avec l'acide 2-méthoxy-4-méthylthiobenzoïque en présence d'oxychlorure de phosphore et de pyridine à une température d'environ 25° C à environ 125° C.

2. Procédé selon la revendication 1, caractérisé en ce qu'on effectue la réaction à une température d'environ 50° C à environ 80° C.

3. Procédé selon la revendication 1, caractérisé en ce qu'on utilise des quantités à peu près équimolaires de la 3,4-diaminopyridine et de l'acide 2-méthoxy-4-méthylthiobenzoïque.